# EUROPEAN PATENT SPECIFICATION

(11) **EP 1 787 672 B1**
(45) Date of publication and mention of the grant of the patent: **02.04.2014**
(21) Application number: 07103642.0
(22) Date of filing: 17.11.1999
(51) Int. Cl.: A61M 16/16, A61M 11/06

(54) **Medical respiratory apparatus**
Medizinisches Atemgerät
Appareil respiratoire médical

(30) Priority: 18.11.1998 GB 9825146
(43) Date of publication of application: 23.05.2007
(62) Divisional of application: 99309138.8
(73) Proprietor: Intersurgical AG, Vaduz (LI)
(72) Inventor: Jassell, Surinderjit Kumar, Windsor, Cheshire SL4 5RQ (GB); Booth, Christopher Edgerly, Ashton, Cheshire CH3 8AB (GB)
(74) Representative: Adamson Jones

(56) References cited:
- EP-A- 0 587 380
- US-A- 3 817 246
- US-A- 3 906 996
- US-A- 4 121 686

## Description

This invention relates to improvements in medical respiratory apparatus of the kind used in patient ventilation, and more particularly to improvements in a combined nebuliser and humidifier.

Patients in hospitals must frequently be provided with an oxygen-enriched air supply, and to avoid drying internal tissue moisture is preferably added to the mixture by means of a nebuliser, which atomises water in a gas jet before supplying the mixture to a face mask. A problem with existing equipment for this purpose is noise emission. This may not be loud but it is irritating to a patient attempting to sleep, for example in an otherwise silent environment, and when numerous nebulisers/humidifiers are in use in the same hospital ward the aggregate noise emission reaches unacceptable levels.

A principal object of the present invention is to address this problem in a simple and economically viable manner.

In accordance with one aspect of a not claimed device there is provided respiratory apparatus for medical use comprising a housing having a first inlet for connection to a source of gas under pressure, second inlet means for the entrainment of air and an outlet for supplying the gaseous mixture to a patient, wherein the interior of the housing is at least partially lined by a tubular baffle of a material having better sound-deadening properties than the material of the housing, the baffle providing a generally funnel-shaped pathway for the mixture between the inlets and the outlet.

The baffle is preferably moulded from a plastics compound of which a greater proportion is an iron ore filler. The compound may have a base of polypropylene and the compound is preferably approximately 60% iron ore to 40% polypropylene. The thickness of the baffle material is preferably in the range 2 to 3mm.

The first inlet may be located in the top of the housing, the outlet may extend radially of the housing between the top and bottom thereof and the second inlet may be disposed radially of the housing between the top thereof and the outlet, the baffle having a generally cylindrical component to line the interior of the housing below the top thereof and a generally trumpet-shaped component coaxial with the cylindrical component and joined thereto at the wider periphery of the trumpet-shaped portion, the narrower-diameter portion of the trumpet-shaped component extending away from the top of the housing beyond the cylindrical component.

A bottom portion of the housing may be a water reservoir from which a tube extends upward through the baffle to a nozzle aligned with gas incoming through the first inlet, thereby to add atomised water to the mixture going to the outlet.

The narrower-diameter portion of the trumpet-shaped component of the baffle may have a radial opening which confronts the interior of the housing at a position substantially diametrically opposite to the outlet.

The junction between the cylindrical and trumpet-shaped components of the baffle may be between top and bottom ends of the cylindrical component and the latter may be cut away where aligned both with the second inlet and with the outlet.

The portion of the cylindrical component which extends below the junction with the trumpet-shaped component may have longitudinal slits in circumferentially spaced relation.

The narrower-diameter portion of the trumpet-shaped component may terminate in a coaxial opening dimensioned closely to surround the water tube.

Another problem associated with existing equipment is that the ring which is turned to adjust the proportion of air admitted to the nebuliser is freely rotatable. It may be accidentally knocked, thereby altering the intended adjustment and this may pass unnoticed.

An example of prior art device is given in US-A-3906996.

Therefore in accordance with an aspect of the present invention there is provided respiratory apparatus for medical use comprising a housing having a first inlet for connection to a source of gas under pressure, second inlet means for the entrainment of air and an outlet for supplying the gaseous mixture to a patient, wherein the second inlet means is adjustable by means of a ring rotatable around the upper part of the housing, the ring being spring loaded to bring a lower end thereof into contact with a shoulder of the housing, said lower end and said shoulder having formations which will inter-engage to prevent rotation of the ring but which may be disengaged to permit rotation of the ring if the ring is lifted against the action of the spring.

The ring is a double-walled structure, closed at one end whereby air is admitted to the second inlet means from an open end of the ring between the walls of the ring, the inner wall of the ring having an opening which can be brought into and out of alignment with the second inlet means of the housing as the ring is rotated.

The second inlet means may comprise a plurality of apertures spaced circumferentially around the housing at the same horizontal level below the top of the housing, and the inner wall of the ring may have a plurality of apertures the circumferential spacing of which corresponds with the circumferential spacing of said plurality of apertures.

A preferred embodiment of the present invention will now be described by way of non-limitative example with reference to the accompanying Drawings, in which:

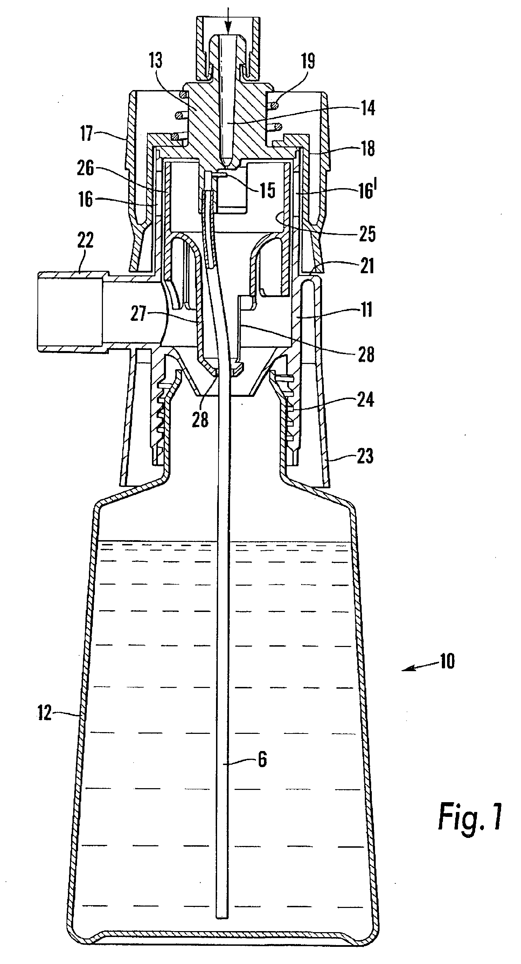

- Figure 1: is a sectional elevation of a nebuliser/humidifier in accordance with the invention;
- Figure 2: is a perspective view of an upper part of the apparatus of Figure 1, partly broken away for purposes of illustration, and
- Figure 3: is a perspective view of the baffle incorporated in the apparatus of Figures 1 and 2.

The nebuliser/humidifier 10 illustrated comprises a housing 11 mounted on a water reservoir 12. At the top of the housing 11 is a first inlet 13 which has standard connections for connecting the interior of the housing to a source of gas under pressure, such as oxygen. As is known *per* se this enters the housing through a nozzle 14 which is at right angles to a nozzle 15 at the upper end of a tube 6 which depends into the water in reservoir 12, so that gas under pressure emerging from nozzle 14 atomises water emerging from nozzle 15 to add water vapour in the form of a fine mist to the gases in the housing.

Below its top the housing 11 has two radial openings or windows 16 and 16' at diametrically opposite positions. These are controlled by rotation of a ring 17 surrounding the upper part of the housing to vary the proportion of air admitted to the interior of housing 11 to mix with gas from the nozzle 14 and water vapour from the nozzle 15.

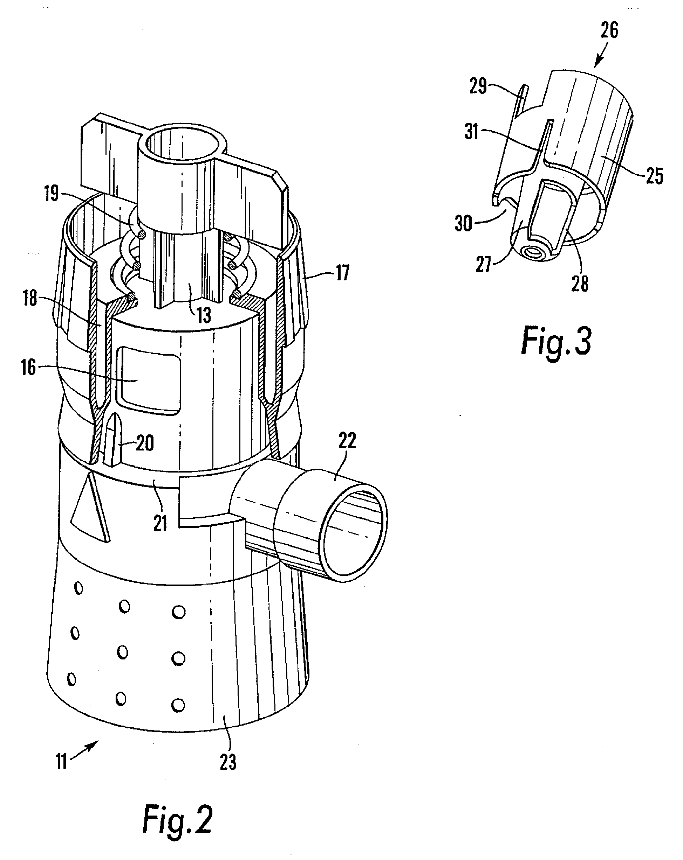

In accordance with one feature of the present invention the ring 17 is a double-walled structure closed at the bottom end of the ring and open at its upper end to admit air between the walls. The inner wall 18 has openings or windows not visible in the Drawings which correspond in position and size to the openings 16,16'. The ring 17 can be rotated between a position in which its windows are fully aligned with the windows 16,16' to admit a maximum volume of air to the interior of the housing 11 and a position in which the windows 16,16' are obturated by the ring 17. A spring 19 biases the ring 17 downward so that lugs, one of which is shown at 20 in Figure 2, which are distributed circumferentially around a shoulder 21 of the housing, may enter recesses (not shown) in the bottom of the ring 17. The engagement of the lugs 20 in these recesses normally prevents rotation of the ring 17 relative to the housing 11, but when it is desired to vary the volume of air admitted to the interior of the housing the ring 17 can be lifted against the action of the spring 19, turned to a different angular position and then released so that the lugs and recesses re-engage with the ring in the new position.

At a radial position below the openings 16,16' the housing 11 has an outlet 22 which is adapted to receive one end of an air line (not shown) the other end of which terminates in equipment such as a face mask (not shown) whereby the mixture of gases and vapour leaving the housing 11 may be administered to a patient.

On a level with and below the outlet 22 the housing 11 has a perforated skirt 23 which in use conceals the screw-threaded connection 24 between the top of the reservoir 12 and the bottom of the housing 11. The housing 11 may be unscrewed from the reservoir 12 to replenish the water in the reservoir.

In accordance with a not claimed device the interior of the housing 11 is lined by a cylindrical component 25 of a baffle 26 which also has a trumpet-shaped component 27 extending below the cylindrical component coaxially within the housing 11. The baffle 26 is of a material having better sound-deadening characteristics than the material from which the housing 11 is made. Specifically the material of the baffle 26 is of greater density than that from which the housing 11 is made. A suitable material for the baffle 26 is a compound of polypropylene and an iron ore filler such as that known as "Magnetite" in proportions of around 60% of the latter. The material of the baffle may have a thickness in the range 2 to 3mm.

The larger-diameter periphery of the trumpet 27 is integral with the cylindrical component 25 at a position about one third the length of the cylindrical component measured from its bottom end. Thus the narrower-diameter part of the trumpet 27 extends downwardly from the cylindrical component 25 coaxially within the housing 11 and terminates at its bottom end in an opening 28 which is just large enough to allow passage through it of the tube 6. The narrower-diameter part of the trumpet also has a much larger radial opening 28 which opens toward the internal wall of the housing 11 diametrically opposite to the outlet 22. Through this opening 28 of the baffle 26 the mixture of gas, air and water vapour is impacted against the interior wall of the housing 11 and then passes around the baffle 26 to exit through the outlet 22.

Notches 29 and 30 in the cylindrical component 25 of the baffle 26 are aligned respectively with the windows 16,16' and with the outlet 22. The lower part of the cylindrical component 25 below its junction with the trumpet-shaped component 27 is formed with four longitudinal slits in circumferentially spaced relation, one of which is visible at 31 in Figure 3.

## Claims

1. Respiratory apparatus for medical use comprising a housing (11) having a first inlet (13) for connection to a source of gas under pressure, second inlet means (16,16') for the entrainment of air and an outlet (22) for supplying the gaseous mixture to a patient, wherein the second inlet means (16,16') is adjustable by means of a ring (17) rotatable around the upper part of the housing (11), the ring (17) being spring loaded to bring a lower end thereof into contact with a shoulder (21) of the housing (11), said lower end and said shoulder (21) having formations (20) which will inter-engage to prevent rotation of the ring (17) but which may be disengaged to permit rotation of the ring (17) if the ring (17) is lifted against the action of the spring (19), **characterized in that** the ring (17) is a double-walled structure, closed at one end whereby air is admitted to the second inlet means (16,16') from an open end of the ring (17) between the walls of the ring (17), the inner wall (18) of the ring (17) having an opening which can be brought into and out of alignment with the second inlet means (16,16') of the housing (11) as the ring (17) is rotated.

2. Apparatus as claimed in Claim 1, wherein the second inlet means comprises a plurality of apertures (16,16') spaced circumferentially around the housing (11) at the same horizontal level below the top of the housing (11), and the inner wall (18) of the ring (17) has a plurality of apertures the circumferential spacing of which corresponds with the circumferential spacing of said plurality of apertures (16,16') of the second inlet means.

3. Apparatus as claimed in Claim 1 or Claim 2, wherein the apparatus includes lugs (20) distributed circumferentially around the shoulder 21 of the housing (11), the lugs (20) being adapted to enter recesses in the lower end of the ring (17).

## Patentansprüche

1. Atemgerät zur medizinischen Verwendung, umfassend ein Gehäuse (11) mit einem ersten Einlass (13) zur Verbindung mit einer Quelle von Gas unter Druck, zweiten Einlassmitteln (16, 16') zur Mitführung von Luft und einem Auslass (22) zum Versorgen eines Patienten mit dem Gasgemisch, wobei das zweite Einlassmittel (16, 16') mithilfe eines Rings (17), der um den oberen Teil des Gehäuses (11) drehbar ist, eingestellt werden kann, der Ring (17) federbelastet ist, um ein unteres Ende davon mit einer Schulter (21) des Gehäuses (11) in Kontakt zu bringen, das untere Ende und die Schulter (21) Ausbildungen (20) aufweisen, die ineinander eingreifen, um Drehung des Rings (17) zu verhindern, aber getrennt werden können, um Drehung des Rings (17) zu ermöglichen, wenn der Ring (17) gegen die Wirkung der Feder (19) angehoben wird, **dadurch gekennzeichnet, dass** der Ring (17) eine doppelwandige Struktur ist, die an einem Ende geschlossen ist, wobei Luft von einem offenen Ende des Rings (17) zwischen den Wänden des Rings (17) in das zweiten Einlassmittel (16, 16') eintreten gelassen wird, wobei die Innenwand (18) des Rings (17) eine Öffnung aufweist, die mit dem zweiten Einlassmittel (16, 16') des Gehäuses (11) in die und aus der Flucht gebracht werden kann, wenn der Ring (17) gedreht wird.

2. Gerät nach Anspruch 1, wobei das zweite Einlassmittel eine Mehrzahl von Öffnungen (16, 16') umfasst, die um das Gehäuse (11) auf der gleichen horizontalen Ebene unter dem oberen Ende des Gehäuses (11) umfänglich beabstandet sind, und die Innenwand (18) des Rings (17) eine Mehrzahl von Öffnungen aufweist, deren umfänglicher Abstand dem umfänglichen Abstand der Mehrzahl von Öffnungen (16, 16') des zweiten Einlassmittels entspricht.

3. Gerät nach Anspruch 1 oder 2, wobei das Gerät Ansätze (20) umfasst, die umfänglich um die Schulter (21) des Gehäuses (11) verteilt sind, wobei die Ansätze (20) so ausgelegt sind, dass sie in Aussparungen im unteren Ende des Rings (17) eintreten.

## Revendications

1. Appareil respiratoire à usage médical comportant un boîtier (11) possédant une première entrée (13) permettant la liaison avec une source de gaz sous pression, un deuxième moyen formant entrée (16, 16') servant à l'introduction d'air et une sortie (22) pour apporter le mélange gazeux à un patient, dans lequel le deuxième moyen formant entrée (16, 16') est réglable au moyen d'un anneau (17) pouvant tourner autour de la partie supérieure du boîtier (11), l'anneau (17) étant sollicité par un ressort pour amener son extrémité inférieure au contact d'un épaulement (21) du boîtier (11), ladite extrémité inférieure et ledit épaulement (21) possédant des formations (20) pouvant s'enclencher mutuellement pour éviter la rotation de l'anneau (17), mais pouvant se désenclencher pour permettre la rotation de l'anneau (17) si l'anneau (17) est soulevé en réaction à l'action du ressort (19), **caractérisé en ce que** l'anneau (17) est une structure à double paroi, fermée à une extrémité, ce par quoi l'air est admis par le deuxième moyen formant entrée (16, 16') à partir d'une extrémité ouverte de l'anneau (17) entre les parois de l'anneau (17), la paroi interne (18) de l'anneau (17) possédant une ouverture pouvant être alignée et désalignée vis-à-vis du deuxième moyen formant entrée (16, 16') du boîtier (11) lorsque l'anneau (17) est mis en rotation.

2. Appareil selon la revendication 1, dans lequel le deuxième moyen formant entrée comporte plusieurs ouvertures (16, 16') espacées sur la circonférence du boîtier (11) au même niveau horizontal au-dessous du sommet du boîtier (11), et la paroi interne (18) de l'anneau (17) possède plusieurs ouvertures dont l'espacement circonférentiel correspond à l'espacement circonférentiel desdites plusieurs ouvertures (16, 16') du deuxième moyen formant ouverture.

3. Appareil selon la revendication 1 ou 2, dans lequel l'appareil comporte des ergots (20) répartis sur la circonférence de l'épaulement (21) du boîtier (11), les ergots (20) étant conçus pour entrer dans des retraits situés dans l'extrémité inférieure de l'anneau (17).
